# FASCICULE DE BREVET EUROPEEN

(11) **EP 4 504 830 B1**
(45) Date de publication et mention de la délivrance du brevet: **25.02.2026**
(21) Numéro de dépôt: 23717890.0
(22) Date de dépôt: 05.04.2023
(51) Int. Cl.: C08J 11/22, C08J 11/24, C08J 11/26

(54) **PROCEDE DE DEPOLYMERISATION DE POLYESTERS TEREPHTALIQUES EN ESTERS DE TEREPHTALATE A TEMPERATURE AMBIANTE**
VERFAHREN ZUR DEPOLYMERISATION VON TEREPHTHALSÄUREPOLYESTERN BEI RAUMTEMPERATUR ZU TEREPHTHALATESTERN
METHOD FOR ROOM TEMPERATURE DEPOLYMERIZATION OF TEREPHTHALIC POLYESTERS TO TEREPHTHALATE ESTERS

(30) Priorité: 06.04.2022 FR 2203148; 19.01.2023 FR 2300523
(43) Date de publication de la demande: 12.02.2025
(73) Titulaire: Recyc'Elit, 69200 Venissieux (FR)
(72) Inventeur: EL MAHDI, Ayoub, 38200 Vienne (FR); MEDIMAGH, Raouf, 69008 Lyon (FR); FOURDIN, Théo, 69003 Lyon (FR)
(74) Mandataire: Plasseraud IP
(86) Numéro de dépôt international: PCT/EP2023/058949
(87) Numéro de publication internationale: WO 2023/194442

(56) Documents cités:
- JP-A- 2004 161 666
- US-B1- 9 550 713
- US-B2- 11 248 103

## Description

La présente invention a trait au domaine du recyclage des matériaux comprenant du polyester de téréphtalate, notamment du polytéréphtalate d'éthylène (PET) ou du polybutylène téréphtalate (PBT) couramment utilisés pour la fabrication de bouteilles en plastiques jetables, barquettes alimentaires, textile, matériau composite pour isolation... Elle concerne notamment un procédé permettant le recyclage du PET en diester téréphtalique et notamment le téréphtalate de diméthyle (DMT) en moins d'une heure et sans étape de prétraitement. De plus, ce procédé ne met pas en œuvre de produit toxique. Il est donc particulièrement avantageux d'un point de vue industriel.

### Domaine de l'invention

Le recyclage du PET est un sujet environnemental important et représente ainsi une opportunité commerciale en raison de son utilisation généralisée, de son abondance et de sa durabilité. Cependant, le recyclage des matières à base de PET est complexe et varie selon le type de polymère, la conception du matériau et le type de produit fini.

Le principal frein à l'utilisation de matières plastiques recyclées est la contamination des flux de déchets avec différents types de polymères qui ne sont pas compatibles les uns avec les autres. Par conséquent, il n'est souvent pas possible d'ajouter du plastique de type PET recyclé au polymère vierge sans diminuer certains attributs de qualité, tels que la couleur, la clarté ou la résistance aux chocs. De ce fait, la capacité de remplacer un polymère vierge par du PET recyclé dépend fortement de la pureté du produit recyclé et des exigences du produit final.

Selon le principe de recyclage chimique, le PET peut être dépolymérisé par une solvolyse telle que la méthanolyse ou la glycolyse, ou par hydrolyse, et les monomères ainsi obtenus peuvent être réutilisés pour générer de nouveaux polymères de PET dit « PET recyclés ».

Selon le besoin industriel, certaines technologies de fabrication de résine PET ont recours à l'utilisation de l'ester diméthanolique de l'acide téréphtalique (DMT).

De plus, les techniques de méthanolyse conventionnelles font appel à des procédés très gourmands en énergie et en coût d'équipements ; ces procédés mettent en œuvre une phase supercritique à des températures supérieures à 300°C et 5 à 10 bars de pression. Du fait des conditions réactionnelles drastiques en termes de températures et de pression, ces technologies induisent car ces induit des changements structuraux des unités moléculaires du PET notamment une isomérisation ou à des dégradations (US 6,706,843 ; WO2021/126661) ; elles ne sont donc pas adaptées à la dépolymérisation de certains matériaux à base de PET comme les matériaux textiles multifibres puisqu'elles conduiraient à la production de molécules de PET modifiées du fait de la « pollution » par des résidus provenant des autres constituants du textile. Ces molécules modifiées peuvent être toxiques ou engendrer des perturbations lors de la production de PET recyclé, elles nuisent à la qualité du produit dépolymérisé pour ces applications futures.

Le document WO2020/128218 décrit un procédé de dépolymérisation du PET par alcoolyse mettant en œuvre un monoalcool tel que le méthanol ou l'éthanol et une base choisie parmi le méthoxyde de sodium, le KOH ou le NaOH en quantité stœchio-métrique par rapport au PET.

Il est connu que l'utilisation d'une base en quantité catalytique par rapport à la masse du PET permet d'obtenir du DMT mais la cinétique de la réaction est assez lente ; le temps de réaction est supérieur à 10h30, temps pendant lequel la solution réactionnelle est chauffée en continu. A titre d'exemple, on peut citer les documents US2019/0256450 et WO2020/188359 qui décrivent la dépolymérisation du PET en DMT en présence de méthanol et d'un alkoxyde tel que le méthoxyde de sodium. Ces réactions de méthanolyse ont lieu à des températures comprises entre 25°C et 100°C. Ces procédés comprennent obligatoirement une première phase de gonflement du PET avec des solvants chlorés, ou polaires tels que le DMSO ou le DMF ou le méthanol. Le document US2019/0256450 propose de faire réagir le PET avec une base, le méthoxyde de sodium en quantité catalytique, et du méthanol. Le procédé décrit dans le document WO2020/188359 se caractérise par l'ajout séquentiel de méthanol et de solutions de méthylate plusieurs fois après ajout de méthoxyde de sodium. Les auteurs décrivent des rendements élevés de production de PET. Le document US2019/390035 décrit une autre approche de dépolymérisation par ajout de sel de glycolate ; la préparation de ce sel comprend des étapes d'isolement et de séchage qui s'étendent sur une semaine.

Le document WO2021/126661 décrit un procédé amélioré de dépolymérisation du PET par méthanolyse en utilisant des catalyseurs choisis parmi le carbonate de sodium, le méthoxyde de magnésium, la DBU et le TBD. Ce procédé est mis en œuvre à des températures au minimum de 110 - 140°C en appliquant une pression de 15 bars.

Le document US 11 248 103 B2 décrit un procédé de recyclage du PET en DMT et éthylène glycol qui utilise un catalyseur conforme à la présente demande et un alcool. Ce procédé n'utilise cependant pas un solvant de type ester ou étheroxyde cyclique.

Pour l'homme du métier, les mises en œuvre des procédés décrits précédemment présentent à l'évidence des problèmes d'opérabilité et de faisabilité industrielles quant à l'aspect sécuritaire d'un environnement ATEX tel que celui du méthanol à reflux qui requiert des précautions complexes et des dispositifs onéreux en vue d'introduire en cours de procédé des produits inflammables.

Aucun de ces procédés n'est satisfaisant. Il est donc souhaitable de disposer de procédés de recyclage de matériaux à base de PET améliorés, à faible coût et facilement opérables industriellement afin de faciliter la généralisation de ce recyclage et d'élargir les champs d'utilisation du PET recyclé, et plus généralement du polyester de téréphtalate.

### Exposé de l'invention

Les inventeurs ont mis au point un nouveau procédé particulièrement performant de dépolymérisation par alcoolyse en conditions douces pour le recyclage de matériaux comprenant du polyester de téréphtalate, notamment du polytéréphtalate d'éthylène (PET) ou du polybutylène téréphtalate (PBT) en monomères d'esters de téréphtalate. Ce procédé est très rapide tout en étant beaucoup plus respectueux de l'environnement que les procédés de l'état de la technique. Il donne accès à un produit sous forme solide directement réutilisable du fait de sa pureté, en particulier du DMT, du DET ou du BHET sous forme cristalline.

Ainsi, l'invention concerne un procédé de recyclage d'un matériau comprenant un polyester de téréphtalate en un ester de téréphtalate comportant deux étapes :
a. une étape de broyage ou déchiquetage dudit matériau pour produire des fragments, et
b. une étape de dépolymérisation du polyester de téréphtalate en ester de téréphtalate, en présence :
   (i) d'un catalyseur choisi parmi une base étheroxyde métallique ou organique, un acétate de métal, un oxyde métallique, un hydroxyde de métal ou un carbonate métallique et
   (ii) d'un solvant polaire du type ester ou étheroxyde cyclique
   (iii) d'un alcool choisi parmi un monoalcool ou d'un diol
   caractérisé en ce que :
   - ladite base est présente en quantité catalytique par rapport à la quantité dudit polyester de téréphtalate
   - ladite étape de dépolymérisation se fait à température ambiante ou en chauffant jusqu'à 70°C pendant une durée comprise entre 1 minute et 4h.

Le matériau à base de polyester de téréphtalate peut être un plastique, un textile ou un autre type de matériau contenant 100% de polyester de téréphtalate (PET ou PBT) ou un matériau composite contenant un mélange de polyester de téréphtalate avec autres constituants tels que le coton, le polyamide, les polyuréthanes, les polyoléfines et les polymères fluorés, tels qu'un plastique composite, un textile multifibre ou un matériau composite d'isolation.

### Avantages de l'invention

Le procédé selon l'invention propose d'associer (i) un catalyseur en quantité catalytique par rapport au polyester de téréphtalate,(ii) un alcool qui est soit un monoalcool, soit un diol et (iii) un solvant du type ester et de les faire réagir dans des conditions douces. Il présente plusieurs avantages au regard des procédés décrits antérieurement, qui sont exposés ci-après.

Un premier avantage remarquable : ce procédé ne nécessite pas de prétraitement, étape qui nécessite la mise œuvre de produits toxiques. La réaction de dépolymérisation est suffisamment efficace pour permettre une dépolymérisation complète sans gonflement préalable de la matière à traiter. Ainsi, le procédé selon l'invention est plus simple (une étape en moins), plus respectueux de l'environnement (pas de produit toxique donc pas d'effluent à traiter), plus rapide et moins cher.

Le procédé étant à risque industriel très modéré, les installations industrielles pour sa mise en œuvre peuvent par conséquent être mises en place plus aisément, le niveau de sécurisation de ces installations étant moins contraignant. La mise en conformité règle-mentaire est simplifiée lors de l'installation de l'usine et tout au long du cycle de production. Le CAPEX est ainsi diminué de manière significative.

Tout type de solvant peut être utilisé pour la dépolymérisation bien que les solvants de type ester soient préférés. Ces derniers sont en effet dénués de toute toxicité, ce sont des produits qui sont notamment utilisés en agroalimentaire, dans le domaine des arômes.

De manière remarquable, la réaction de dépolymérisation est complète, très rapide et produit un ester de téréphtalate d'une grande pureté. Ceci est applicable aussi bien pour le PET que pour le PBT qui sont dépolymérisés en DMT, DET ou BHET. Ces derniers sont alors facilement recyclables et ont des débouchés industriels et un marché reconnu.

Le procédé peut être qualifié de « très rapide » puisque que la réaction est complète en moins de 4h à température ambiante, et en moins de 20 minutes en conditions de chauffage optimisées, notamment entre 55 et 70°C. Elle démarre instantanément et peut conduire à une dépolymérisation complète dès 1 min.

La réaction de dépolymérisation est simple. La dépolymérisation et la purification peuvent se faire en une seule et même étape. Après complétion de la réaction, le produit obtenu est directement un ester de téréphtalate sous forme de cristaux. Un lavage permet d'enlever les produits intermédiaires ou de dégradation qui nécessiteraient des opérations de distillation fastidieuses dans les procédés classiques afin de les séparer du produit d'intérêt.

Le procédé permet ainsi d'obtenir du DMT, du DET ou du BHET selon que la dépolymérisation du PET ou du PBT est effectuée par méthanolyse, éthanolyse ou glycolyse respectivement.

Ce procédé peut être appliqué à tout type de matériau comprenant un polyester de téréphtalate, en particulier du PET, ou du PBT, pur ou en mélange, transparent ou coloré, quelle que soit son épaisseur ou sa composition.

L'homme du métier sait que les matériaux textiles composites et multifibres peuvent être fabriqués de différentes manières. Ils peuvent être tissés et enduits sous forme de plusieurs couches, ou de nature non tissée. Ils sont composés de différents matériaux en particulier de polyester mélangé à d'autres matériaux.

La nature des matériaux de type « polyester » peut différer et à titre non exhaustif ce dernier peut être du Polyéthlène téréphtalate, (PET); du Polybutylène téréphtalate (PBT), de l'acide polylactique (PLA), un polycaprolactone (PCL)... Les matériaux pouvant être recyclés grâce au procédé selon l'invention comprennent au moins un polyester téréphtalique de type PET ou PBT.

Les autres matériaux mélangés au polyester peuvent être, de manière non limitative, le polyamide (Nylon 6,6 ou diadipate d'hexamethylne diamine, Nylon 6 ou polyca-prolactame...), les polyuréthanes, le coton ; des polyoléfines (Polypropylène, Polyéthylène), des polymères fluorés (Ces derniers sont généralement enduits. Les polymères fluorés sont utilisés pour leurs propriétés d'étanchéité et d'isolation ; à titre exemple, on peut citer le Polytetrafluoroéthylène (PTFE). Parmi les polyuréthanes, l'élasthanne est particulièrement apprécié car il apporte une souplesse au textile et des propriétés respirantes notamment lorsqu'il s'agit de polyuréthane élastomère souple dont les deux principales formes commercialisées sont les poly(ether)uréthanes et les poly(ester)uréthanes.

L'utilisation des polyamides en des proportions variables avec le PET trouve beaucoup d'applications dans le domaine de l'habillement (lingerie et vêtement de sport) mais également dans les textiles techniques pour leurs propriétés hautement absorbantes et sans poussières (lingettes microfibres pour essuyage industriel). Toutefois, il n'est actuellement pas possible de recycler un matériau composite comprenant de l'élasthanne, des polyamides ou des matériaux enduits, ce qui est problématique en termes de gestion des déchets ; le procédé selon l'invention apporte une solution à ce problème.

De plus, le procédé selon l'invention est particulièrement intéressant pour le recyclage de matériau composite à base de polyester de téréphtalate du fait que la réaction est sélective vis-à-vis du polyester de téréphtalate et ne modifie pas les autres composants éventuels ; la séparation est ainsi aisée entre le polyester de téréphtalate dépolymérisé sous forme de monomères et les autres composants ; ces derniers peuvent être récupérés par simple filtration et lavage. Ensuite - après une éventuelle étape de décoloration dans le cas du textile - le mélange refroidi permet la précipitation des monomères d'esters de téréphtalate. Un lavage suffit pour obtenir du DMT, du DET ou du BHET directement utilisable. Les solvants de lavage sont avantageusement les alcools utilisés lors de la dépolymérisation.

Un autre avantage différenciant du procédé est qu'il permet de valoriser les matériaux séparés du polyester de téréphtalate après la dépolymérisation de ce dernier, sous une forme non altérée. Le polyamide (polyamide 6 ; polyamide 6,6), l'élasthanne, le coton en sont des exemples concrets, dans le domaine du textile. Le procédé décrit dans ce brevet permet d'isoler les composants initialement mélangés au polyester de téréphtalate avec un degré de pureté permettant leur recyclage ultérieur. On peut noter que l'une des applications très innovantes de ce procédé est de permettre la récupération de l'élasthanne ou du polyamide à partir de matériau composite à base de polyester de téréphtalate et sa réutilisation dans de nouvelles applications.

Le rendement du procédé est élevé : au moins de 85% notamment pour la dépolymérisation du PET en DMT. De plus, la matière mélangée au polyester est restituée dans son intégralité.

Dans le cas particulier de la dépolymérisation du PET en DMT en utilisant du méthanol, le produit obtenu est pur à 99,9% en fin de réaction (après filtration et lavage) ; il n'y a donc pas besoin de purification ultérieure. Le DMT peut être utilisé directement après lavage au méthanol. Compte tenu de son niveau de pureté, il peut être utilisé dans de nombreuses applications, pour refaire du PET ou tout autre type de résine technique faisant intervenir ce monomère. Le choix des réactifs et la mise en œuvre de conditions douces font qu'aucune réaction d'isomérisation ne se produit, ni la formation de produits de dégradation qui nuisent à la qualité du produit obtenu. Lorsqu'elles sont présentes, ces molécules secondaires à la réaction perturbent la réaction de polymérisation et une purification du DMT brut est donc nécessaire avant son utilisation. Ceci est généralisable à la dépolymérisation du PET et du PBT en monomères d'esters de téréphtalate de tout type (DMT, DET et BHET).

Ce procédé est plus économique et plus respectueux de l'environnement que les procédés existants du fait que les bases (catalyseurs) sont utilisées dans des quantités catalytiques par rapport à la quantité de polyester de téréphtalate à recycler, et que les températures de réactions sont inférieures à 80°C, généralement comprises entre la température ambiante (autour de 25°C) et 60°C et que les temps de réaction sont très réduits par rapport à ceux des procédés de dépolymérisation du PET décrits dans la littérature.

En particulier, l'alcool est utilisé dans des proportions allant de 0,25 à 16 équivalents molaires par rapport au polyester de téréphtalate ; préférentiellement de 0,6 à 9 équivalents molaires par rapport au polyester de téréphtalate ; plus précisément de 1,1 à 4,9 équivalents molaires par rapport au polyester de téréphtalate, ce qui est une amélioration substantielle par rapport aux technologies de méthanolyse classique dans lesquelles des proportions de 25 fois molaires sont nécessaires.

Les proportions du solvant polaire de type acétate sont également réduites allant au minimum de 1 : 1,5 en ce qui concerne la masse de polyester de téréphtalate : volume du mélange de solvants allant à un ratio 1 : 10 pour ce qui est de la masse de polyester de téréphtalate : volume de mélange de solvants.

D'un point de vue écologique, il est à noter que le bain de dépolymérisation contenant le solvant peut être réutilisé pour un nouveau cycle de traitement une fois le produit filtré. Le bain peut être utilisé au moins 2 fois sans affecter l'efficacité de la réaction. Une fois la réaction finie, les solvants peuvent être récupérés par simple distillation peu énergétique étant donné leur faible point d'ébullition.

### DESCRIPTION DETAILLEE DE L'INVENTION

L'invention concerne un procédé de recyclage d'un matériau comprenant un polyester de téréphtalate en ester de téréphtalate comportant deux étapes :
a. une étape de broyage ou de déchiquetage des déchets pour produire des fragments, et
b. une étape de dépolymérisation dudit polyester en ester de téréphtalate en présence :
   (i) d'un catalyseur choisi parmi une base étheroxyde métallique ou organique, un acétate de métal, un oxyde métallique, un hydroxyde de métal, un carbonate métallique ou un ester métallique, et
   (ii) d'un solvant polaire du type ester ou étheroxyde cyclique
   (iii) d'un alcool choisi parmi un monoalcool ou d'un diol

   caractérisé en ce que :
      - ladite base est présente en quantité catalytique par rapport à la quantité dudit polyester
   ladite étape de dépolymérisation se fait à température ambiante ou en chauffant jusqu'à 70°C pendant une durée comprise entre 1 minute et 4h.

Dans un mode de réalisation préféré, l'invention concerne un procédé de recyclage de matériaux comprenant du polyéthylène téréphtalate en monomères esters de téréphtalate comportant deux étapes :
a. une étape de broyage ou de déchiquetage des déchets pour produire des fragments, et
b. une étape de dépolymérisation du PET en ester de téréphtalate et monoéthylène glycol (MEG), en présence :
   (i) d'un catalyseur choisi parmi une base étheroxyde métallique ou organique, un acétate de métal, un oxyde métallique, un hydroxyde de métal, un carbonate métallique ou un ester métallique, et
   (ii) d'un solvant polaire du type ester ou étheroxyde cyclique
   (iii) d'un alcool choisi parmi un monoalcool ou d'un diol
   caractérisé en ce que :
   - ladite base est présente en quantité catalytique par rapport à la quantité de PET
   - ladite étape de dépolymérisation se fait à température ambiante ou en chauffant jusqu'à 70°C pendant une durée comprise entre 1 minute et 4h.

Il est connu de l'homme du métier que la dépolymérisation d'un polyester de téréphtalate produit du DMT et un diol correspondant au polyester, à savoir du monoéthylène glycol à partir de PET et du butane diol à partir du PBT.

Les matériaux comprenant du polyester de téréphtalate peuvent être composés à 100% de polyester de téréphtalate (par exemple des plastiques ou des textiles) ou être composés d'un mélange comprenant du polyester de téréphtalate et d'autres composants tels que le coton, le polyamide, l'élasthanne, le PTFE, le polyéthylène, le polypropylène (par exemple des plastiques composites, des textiles multifibres ou de panneaux composites d'isolation).

Lorsque le matériau comprenant du polyester de téréphtalate, est constitué à 100% de polyester de téréphtalate, ce matériau est transformé en ester de téréphtalate. Celui-ci peut être récupéré par simple filtration et précipité par refroidissement, comme cela est décrit après et illustré dans la partie expérimentale.

Le polyester de téréphtalate est choisi par le parmi le polyéthylène téréphthalate (PET) et le polybutylène téréphtalate (PBT).

Lorsque le matériau comprenant du polyester de téréphtalate est un matériau composite comprenant du polyester de téréphtalate mélangé à d'autres composants, le procédé de recyclage produit des esters de téréphtalates (DMT) par dépolymérisation du polyester de téréphtalate, les autres composants du matériau se trouvant sous une forme non altérée dans le mélange réactionnel. Ces autres composants étant de taille supérieure à celle du DMT, ils peuvent être séparés de ce dernier par simple filtration.

Le matériau composite comprend du polyester de téréphtalate choisi parmi le polyéthylène téréphthalate et le polybutylène téréphtalate, mélangés à un autre constituant choisi parmi le coton, le polyamide, les polyuréthanes, les polyoléfines et les polymères fluorés.

Ainsi, dans un mode de réalisation particulier dans lequel le matériau comprenant du PET est un matériau composite comprenant du PET mélangé à d'autres composants, le procédé de recyclage permet la transformation (recyclage) du PET en DMT et la libération des autres composants (qui peuvent également être recyclés).

Le catalyseur est une base choisie parmi :
(i) un étheroxyde est du type méthylate de sodium, méthylate de magnésium, méthylate de potassium ou méthylate d'ammonium,
(ii) un carbonate métallique de type carbonate de sodium ou carbonate de potassium,
(iii) un hydroxyde métallique de type hydroxyde de sodium ou hydroxyde de potassium, et
(iv) un acétate de métal de type acétate de zinc Zn(OAc)₂ ou acétate de sodium NaOAc, ou un acétate de potassium KOAc
(v) un oxyde métallique,
(vi) un ester métallique de type ester de titane Ti(OiPr)₄,ester de manganèse Mn(OR)_{2,} ou ester d'antimoine Sb(OR)₂.

Dans un mode de réalisation préféré de l'invention, le catalyseur est choisi parmi le méthylate de sodium, le méthylate de magnésium, le méthylate de potassium ou le méthylate d'ammonium.

Le catalyseur est présent dans un rapport molaire inférieur à 35% par rapport au polyester de téréphtalate de préférence compris entre 1 et 20%.

Le solvant de type ester peut être un monoester, un diester ou un triester.

Le solvant de type ester répond de préférence à la formule A :

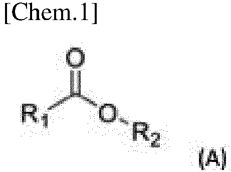

Le solvant du type étheroxyde répond de préférence à la formule B : 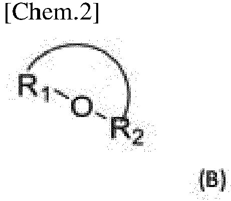 dans lesquelles R₁ et R₂ sont identiques ou différents et sont choisis (indépendamment) parmi un aryl CₙH₂ₙ,alkyl CₙH₂ₙ₊₁ouCₙH₂ₙ₋₁ avec n = 1 à 10.

Dans un mode de réalisation préféré, le solvant polaire est de type ester car non toxique. Le solvant de type ester peut-être choisi parmi l'acétate de méthyle, d'éthyle, propyl, butyl, isopropyle.

Le Tableau 2 (partie expérimentale) décrit différents modes de réalisation de l'invention en fonction de la base utilisée.

Le solvant peut aussi être de type éthéroxyde cyclique, comme le dioxane.

Dans un mode de réalisation préféré de l'invention, le ratio polyester de téréphtalate : solvant est compris entre 1 : 1,5 et 1 : 10.

La quantité d'alcool mise en jeu dans la réaction de dépolymérisation est variable. L'alcool peut être soit apporté par la base en solution (dans un alcool), soit est ajouté en tant que tel dans le milieu réactionnel. L'alcool peut ainsi être en excès, en quantité équivalente ou en défaut par rapport à la quantité de polyester de téréphtalate. Ce paramètre sera ajusté par l'homme du métier.

L'alcool est présent dans un ratio compris entre 0,25 et 16 équivalents molaires par rapport au polyester de téréphtalate. Dans un autre mode de réalisation préféré de l'invention, le ratio molaire alcool : polyester de téréphtalate est compris entre 0,5 et 16 ; préférentiellement entre 0,6 et 9 ; plus préférentiellement précisément entre 1,1 et 3.

De manière avantageuse, le procédé est mis en œuvre en appliquant un ratio polyester de téréphtalate : mélange de solvant compris entre 1 : 1,5 et 1 : 10 et un ratio molaire alcool : polyester de téréphtalate compris entre 0,25 et 10. Dans un mode de réalisation particulier, le procédé est mis en œuvre en appliquant un ratio polyester de téréphtalate : mélange de solvant compris entre 1 : 1,5 et 1 : 5 et un ratio molaire alcool : polyester de téréphtalate compris entre 0,25 et 3.

L'alcool mis en œuvre pendant l'étape de dépolymérisation est de préférence un monoalcool choisi parmi le méthanol, l'éthanol, le propanol ou le butanol, ou un diol tel que l'éthylène glycol.

Dans un mode de réalisation particulier de l'invention, on utilise un alcool et un ester de même rang lors de la réaction de dépolymérisation.

Cette combinaison d'un alcool et d'un ester de même rang a pour avantage de permettre une réaction de dépolymérisation complète. Les monomères de téréphtalate sont ainsi solubilisés. Il suffit de refroidir la solution pour les précipiter et récupérer un produit d'une grande pureté (au moins 99 %).

Si le matériau comporte un mélange de polyester de téréphtalate et d'autres composants, ces derniers ne seront pas modifiés, resteront en suspension et seront facilement éliminés par filtration.

A titre d'exemple, on peut combiner le méthanol et l'acétate de méthyle, on obtient du DMT (réaction de méthanolyse) ou l'éthanol et l'acétate d'éthyle, on obtient du DET (diester de téréphtalate diéthylique) (réaction d'éthanolyse). Si l'on utilise du diéthylène glycol, on obtient du BHET (bis(2-Hydroxyethyl) téréphthalate) (réaction de glycolyse).

L'intérêt du DET est par exemple illustré dans le document WO2007/076384 qui décrit une réaction d'éthanolyse du PET. La production de DET est décrite comme avantageuse le fait que le DET est plus facile à dissoudre que le DMT. Le DET obtenu peut être oxydé puis servir à produire de l'acide téréphtalique.

Alternativement, un autre mode de réalisation selon l'invention peut consister à combiner un alcool et un ester de rang différent. On peut par exemple combiner de l'acétate d'éthyle et du méthanol, deux réactifs d'usage commun. La réaction de dépolymérisation du PET se déroule de manière efficace et complète et l'on obtient un produit majoritaire correspondant à l'alcool utilisé, dans cet exemple du DMT du fait de la présence de méthanol, mais aussi des produits secondaires comme du DET et autres monomères de téréphtalate.

La base mise en jeu dans la réaction de dépolymérisation peut être en quantité catalytique par rapport à la quantité de polyester de téréphtalate à traiter.

Par « quantité catalytique », on entend une quantité de base non-stœchiométrique, c'est-à-dire dans un rapport molaire de 1% à 49% par rapport à la quantité de polyester de téréphtalate à traiter. Le terme « catalytique » s'applique également à un réactif que l'on retrouve dans sa forme initiale en fin de réaction (catalyseur).

Dans un mode de réalisation préféré de l'invention, la quantité catalytique de base éthéroxyde est inférieure à 35% molaire. La quantité catalytique de base éthéroxyde peut varier de 1 % molaire à 35 % molaire, de préférence de 1% à 20% molaire, voire de 5% à 20%. Des temps de réaction prolongés peuvent être appliqués afin de diminuer encore cette quantité, ce qui permet de réduire le coût de la réaction.

La température de réaction peut varier. Le milieu réactionnel peut être chauffé jusqu'à 70°C. Le mélange peut notamment être avantageusement chauffé entre 50°C et 70°C, de préférence à une température inférieure à 60°C. Il est toutefois très intéressant de noter que la réaction fonctionne très bien à température ambiante (autour de 25°C) tout en étant rapide puisqu'une dépolymérisation complète est obtenue en 3 à 4h. Le fait de ne pas chauffer la réaction simplifie la mise en œuvre et réduit le coût.

La présente invention sera mieux comprise à la lecture des exemples qui suivent, fournis à titre d'illustration et ne devant en aucun cas être considérés comme limitant la portée de la présente invention.

### PARTIE EXPERIMENTALE

### EXEMPLE 1 : Dépolymérisation du PET par méthanolyse

Une quantité (500 g) de morceaux de polyéthylènetéréphtalate (PET) provenant de différentes sources (barquettes alimentaires, bouteilles d'eau...) est introduite dans 2L d'acétate de méthyle. 120 mL d'une solution de méthoxyde de sodium (25 % dans le méthanol) correspondant à un rapport molaire de 20 % de méthoxyde de sodium par rapport au PET introduit et 200 mL de méthanol sont ajoutés aux morceaux. La réaction démarre instantanément. Au bout de 30 minutes de réaction à 55 °C, la totalité des morceaux de PET a disparu laissant place à un solide blanc légèrement suspendu en solution. Le brut réactionnel est filtré sur Buchner afin de retenir la matière non réagie, le milieu récupéré gélifie quasi instantanément. Il contient le DMT, le monoéthylène glycol produit de la réaction de dépolymérisation ainsi que la base mise en réaction initialement et le solvant. Le solide blanc (DMT) qui est récupéré (410 g, 82%) et est lavé par du méthanol.

### EXEMPLE 2 : Dépolymérisation du PET par méthanolyse

Une quantité (500 g) de morceaux de PET provenant de différentes sources (barquettes alimentaires, bouteilles d'eau...) est introduite dans 2L d'acétate de méthyle. 210 mL d'une solution de méthoxyde de sodium (25 % dans le méthanol) correspondant à un rapport molaire de 35 % de méthoxyde de sodium par rapport au PET introduit sont ajoutés aux morceaux. La réaction démarre instantanément. Au bout de 30 minutes de réaction à 55 °C, la totalité des morceaux de PET a disparu laissant place à un solide blanc légèrement suspendu en solution. Le brut réactionnel est filtré sur Buchner afin de retenir la matière non réagie, le milieu récupéré gélifie quasi instantanément. Il contient le DMT, le monoéthylène glycol produit de la réaction de dépolymérisation ainsi que la base mise en réaction initialement et le solvant. Le solide blanc (DMT) qui est récupéré (400 g, 80 %) et est lavé par du méthanol.

### EXEMPLE 3 : Dépolymérisation du textile 100 % PET par méthanolyse

Une quantité (500 g) de morceaux de textile 100% PET colorés est introduite dans 2L d'acétate de méthyle. 119 mL d'une solution de méthoxyde de sodium (25 % dans le méthanol) correspondant à un rapport molaire de 20 % de méthoxyde de sodium par rapport au PET introduit et sont ajoutés aux morceaux. La réaction démarre instantanément. Au bout de 120 minutes de réaction à 55 °C, la totalité des morceaux de PET est dépolymérisée laissant place à un solide coloré légèrement suspendu en solution. Une étape de décoloration a été réalisée par l'ajout du charbon actif afin d'avoir un DMT blanc. Le brut réactionnel est filtré sur Buchner afin de retenir la matière non réagie et le charbon actif, le milieu récupéré gélifie quasi instantanément. Il contient le DMT, le monoéthylène glycol produit de la réaction de dépolymérisation ainsi que la base mise en réaction initialement et le solvant. Le solide blanc (DMT) qui est récupéré (350 g, 70 %) et est lavé par du méthanol.

### EXEMPLE 4 : Dépolymérisation d'un matériau textile en mélange PET/Élasthanne 85%/15% par méthanolyse

Une quantité (500 g) de morceaux de textile mélange PET/Élasthanne composé à hauteur de 85% PET et 15 % Élasthanne colorés contenant différents pourcentages en Élasthanne est introduite dans 3.2L d'acétate de méthyle et 0.8L de méthanol. 119 mL d'une solution de méthoxyde de sodium (25 % dans le méthanol) correspondant à un rapport molaire de 20 % de méthoxyde de sodium par rapport au textile introduit sont ajoutés aux morceaux. La réaction démarre instantanément. Au bout de 80 minutes de réaction à 55 °C, la totalité des morceaux de textile à base de PET ont été dépolymérisés laissant place aux morceaux d'élasthanne non réagis et un solide coloré légèrement suspendu en solution. Une étape de préfiltration a été réalisée pour retenir l'élasthanne (75g, 15%) et la matière non réagie. L'étape suivante est la décoloration en ajoutant le charbon actif sur le brut réactionnel afin d'avoir un DMT blanc. Le brut réactionnel est filtré sur Buchner afin de retenir le charbon actif, le milieu récupéré gélifie quasi instantanément. Il contient le DMT, le monoéthylène glycol produit de la réaction de dépolymérisation ainsi que la base mise en réaction initialement et le solvant. Le solide blanc (DMT) qui est récupéré (280 g, 66 %) et est lavé par du méthanol.

### EXEMPLE 5 : Dépolymérisation du textile mélange PET/Cotton (80%/20%) par méthanolyse

Une quantité (500 g) de morceaux de textile mélange PET/Coton composé à hauteur de 80% PET et 20 % Coton colorés est introduite dans 5L d'acétate de méthyle. 119 mL d'une solution de méthoxyde de sodium (25 % dans le méthanol) correspondant à un rapport molaire de 20 % de méthoxyde de sodium par rapport au textile introduit sont ajoutés aux morceaux. La réaction démarre instantanément. Au bout de 210 minutes de réaction à 55°C, la majorité des morceaux de textile PET a dégradé laissant place aux morceaux de coton non réagis et un solide coloré légèrement suspendu en solution. Une étape de préfiltration a été réalisée pour retenir le coton (165g, 20%) et la matière non réagie. L'étape suivante est la décoloration en ajoutant le charbon actif sur le brut réactionnel afin d'avoir un DMT blanc. Le brut réactionnel est filtré sur Buchner afin de retenir le charbon actif, le milieu récupéré gélifie quasi instantanément. Il contient le DMT, le monoéthylène glycol produit de la réaction de dépolymérisation ainsi que la base mise en réaction initialement et le solvant. Le solide blanc (DMT) qui est récupéré (245 g, 61 %) et est lavé par du méthanol.

### EXEMPLE 6 : Dépolymérisation du textile mélange PET/Polyamide (90%/10%) par méthanolyse

Une quantité (500 g) de morceaux de textile mélange PET/PA colorés est introduite dans 5L d'acétate de méthyle. 119 mL d'une solution de méthoxyde de sodium (25 % dans le méthanol) correspondant à un rapport molaire de 20 % de méthoxyde de sodium par rapport au textile introduit sont ajoutés aux morceaux. La réaction démarre instantanément. Au bout de 120 minutes de réaction à 55 °C, la totalité des morceaux de textile a dégradé laissant place aux morceaux de PA non réagis et un solide coloré légèrement suspendu en solution. Une étape de préfiltration a été réalisée pour retenir le PA (50g, 10%) et la matière non réagie. L'étape suivante est la décoloration en ajoutant le charbon actif sur le brut réactionnel afin d'avoir un DMT blanc. Le brut réactionnel est filtré sur Buchner afin de retenir le charbon actif, le milieu récupéré gélifie quasi instantanément. Il contient le DMT, le monoéthylène glycol produit de la réaction de dépolymérisation ainsi que la base mise en réaction initialement et le solvant. Le solide blanc (DMT) qui est récupéré (350 g, 70 %) et est lavé par du méthanol.

### EXEMPLE 7 : Dépolymérisation du panneau composite pour isolation 100 % PET par méthanolyse

Une quantité (500 g) de morceaux de mousse 100% PET blancs est introduite dans 1,5L d'acétate de méthyle. 71.43 mL d'une solution de méthoxyde de sodium (25 % dans le méthanol) correspondant à un rapport molaire de 12 % de méthoxyde de sodium par rapport au PET introduit sont ajoutés aux morceaux. La réaction démarre instantanément. Au bout de 20 minutes de réaction à 55°C, la totalité des morceaux de panneau d'isolation a dégradé laissant place à un solide jaune clair légèrement suspendu en solution. Le brut réactionnel est filtré sur Buchner afin de retenir la matière non réagie, le milieu récupéré gélifie quasi instantanément. Il contient le DMT, le monoéthylène glycol produit de la réaction de dépolymérisation ainsi que la base mise en réaction initialement et le solvant. Le solide blanc (DMT) qui est récupéré (360 g, 72 %) et est lavé par du méthanol.

### EXEMPLE 8 : Dépolymérisation du PET par éthanolyse

Une quantité (500 g) de morceaux de PET provenant de différentes sources (barquettes alimentaires, bouteilles d'eau...) est introduite dans 2L d'acétate d'éthyle. 28.12 g de méthoxyde de sodium correspondant à un rapport molaire de 20 % de méthoxyde de sodium par rapport au PET introduit et 300 mL d'éthanol sont ajoutés aux morceaux. La réaction démarre instantanément. Au bout de 30 minutes de réaction à 70°C, la totalité des morceaux de PET a disparu laissant place à un solide blanc légèrement suspendu en solution. Le brut réactionnel est filtré sur Buchner afin de retenir la matière non réagie, le milieu récupéré contient le DET, le monoéthylène glycol produit de la réaction de dépolymérisation ainsi que la base mise en réaction initialement et le solvant. Le DET (400 g) est récupéré sous forme de solide pâteux suite à l'évaporation des solvants réactionnels et est lavé par de l'éthanol.

### EXEMPLE 9 : Taux de conversion en fonction du temps et de la température

Le tableau 1 présente l'effet du temps de réaction et de la température sur le taux de conversion du PET en DMT.

Les conditions réactionnelles mises en œuvre sont les suivantes : 10 g de PET sont incubés dans une solution de méthylate de sodium (diluée à 25 % dans MeOH) dans un ratio de 20 % (mol : mol PET), en présence de 45ml d'acétate de méthyle.

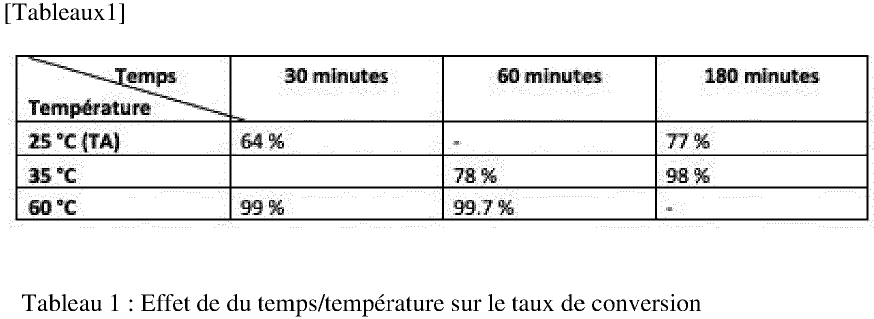

EXEMPLE 10 : Taux de conversion en fonction du type de solvant et d'alcool

Le tableau 2 présente l'effet du temps de réaction et de la température sur le taux de conversion du PET en DMT.

Les conditions réactionnelles sont les mêmes que celles de l'Exemple 4.

Le solvant de type ester répond à la formule ci-dessous :

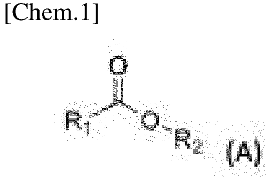

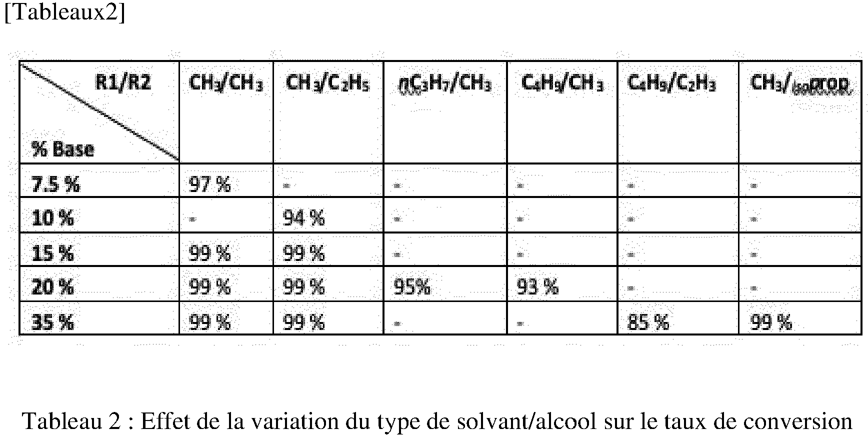

## Revendications

1. Procédé de recyclage d'un matériau comprenant du polyester de téréphtalate en diester de téréphtalate comportant deux étapes :
a. une étape de broyage ou de déchiquetage dudit matériau pour produire des fragments, et
b. une étape de dépolymérisation dudit polyester en ester de téréphtalate en présence :
(i) d'un catalyseur choisi parmi une base étheroxyde métallique ou organique, un acétate de métal, un oxyde métallique, un hydroxyde de métal, un ester métallique ou un carbonate métallique,
(ii) d'un solvant polaire du type ester ou étheroxyde cyclique
(iii) d'un alcool choisi parmi un monoalcool ou d'un diol **caractérisé en ce que** :
- ladite base est présente en quantité catalytique par rapport à la quantité dudit polyester
- ladite étape de dépolymérisation se fait à température ambiante ou en chauffant jusqu'à 70°C pendant une durée comprise entre 1 minute et 4h.

2. Procédé selon la revendication 1 dans lequel ledit matériau est composé à 100% de polyester de téréphtalate choisi parmi le polyéthylène téréphtalate (PET) et le polybutylène téréphtalate (PBT).

3. Procédé selon la revendication 1 dans lequel ledit matériau est un matériau composite comprenant du polyester de téréphtalate choisi parmi le polyéthylène téréphtalate et le polybutylène téréphtalate mélangé à un autre constituant choisi parmi le coton, le polyamide, les polyuréthanes, les polyoléfines et les polymères fluorés.

4. Procédé selon la revendication 3 dans lequel ledit polyuréthane est de l'élasthanne.

5. Procédé selon l'une des revendications 1 à 4 dans lequel ledit catalyseur est choisi parmi (i) un étheroxyde est du type méthylate de sodium, méthylate de potassium, méthylate de magnésium ou méthylate d'ammonium (ii) un carbonate métallique de type carbonate de sodium ou carbonate de potassium, (iii) un hydroxyde métallique de type hydroxyde de sodium ou hydroxyde de potassium, (iv) un acétate de métal type acétate de zinc ou acétate de sodium ou acétate de potassium (v) un oxyde métallique (iv) ou un ester métallique ou (i) un ester métallique de type ester de titane.

6. Procédé selon la revendication 5 dans lequel ledit catalyseur est un ester métallique choisi parmi l'ester de titane, l'ester de manganèse, l'ester d'antimoine, ou l'acétate de zinc, l'acétate de sodium ou l'acétate de potassium.

7. Procédé selon l'un des revendications précédentes dans lequel ledit catalyseur est présent dans un rapport molaire inférieur à 35% par rapport au polyester de téréphtalate.

8. Procédé selon l'une des revendications précédentes dans lequel ledit solvant polaire répond à l'une des formules A ou B : 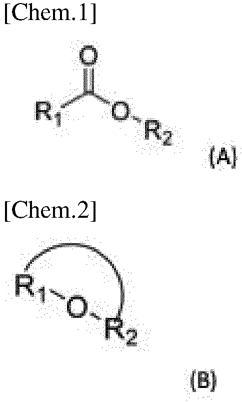 dans lesquelles R₁ et R₂ sont identiques ou différents et sont choisis parmi un aryl CₙH₂ₙ,alkyl CₙH₂ₙ₊₁ouCₙH₂ₙ₋₁ avec n = 1 à 10.

9. Procédé selon la revendication 8 dans lequel ledit ester est choisi parmi l'acétate de méthyle, d'éthyle, propyl, butyl, isopropyle, glycol.

10. Procédé selon l'une des revendications précédentes dans lequel le ratio polyester de téréphtalate: solvant est compris entre 1 : 1,5 et 1 : 10.

11. Procédé selon l'une des revendications précédentes dans lequel ledit monoalcool est choisi parmi le méthanol, l'éthanol, le propanol ou le butanol et ledit diol est l'éthylène glycol.

12. Procédé selon l'une des revendications précédentes dans lequel le ratio (équivalent) molaire alcool : polyester de téréphtalate est compris entre 0,25 et 16.

13. Procédé selon l'une des revendications précédentes dans lequel sont utilisés pour une même réaction de dépolymérisation un alcool et un ester de même rang.

## Patentansprüche

1. Verfahren zum Recycling eines Materials, das Terephthalat- Polyester als Terephthalatdiester aufweist, umfassend zwei Schritte:
a. einen Schritt der Zerkleinerung oder des Schreddern des Materials, um Fragmente zu erzeugen, und
b. einen Schritt der Depolymerisation des Polyesters in Terephthalatester in Gegenwart von:
(i) einem Katalysator, gewählt aus einer metallischen oder organischen Etheroxidbase, einem Metallacetat, einem Metalloxid, einem Metallhydroxid, einem Metallester oder einem Metallcarbonat,
(ii) einem polaren Lösungsmittel vom Ester- oder cyclischen Etheroxidtyp,
(iii) einen Alkohol, gewählt aus einem Monoalkohol oder einem Diol,
**dadurch gekennzeichnet, dass**:
- die Basis in einer katalytischen Menge im Verhältnis zur Menge des Polyesters vorliegt,
- der Depolymerisationsschritt bei Raumtemperatur oder durch Erhitzen auf 70 °C für einen Zeitraum zwischen 1 Minute und 4 Stunden durchgeführt wird.

2. Verfahren nach Anspruch 1, wobei das Material zu 100 % aus Polyester-Terephthalat besteht, gewählt aus Polyethylenterephthalat (PET) und Polybutylenterephthalat (PBT).

3. Verfahren nach Anspruch 1, wobei das Material ein Verbundmaterial ist, das Polyester-Terephthalat, gewählt aus Polyethylenterephthalat und Polybutylenterephthalat, gemischt mit einem anderen Bestandteil, gewählt aus Baumwolle, Polyamid, Polyurethanen, Polyolefinen und fluorierten Polymeren, umfasst.

4. Verfahren nach Anspruch 3, wobei das Polyurethan Elasthan ist.

5. Verfahren nach einem der Ansprüche 1 bis 4, wobei der Katalysator gewählt ist aus (i) einem Etheroxid vom Typ Natriummethylat, Kaliummethylat, Magnesiummethylat oder Ammoniummethylat, (ii) einem Metallcarbonat vom Typ Natriumcarbonat oder Kaliumcarbonat, (iii) einem Metallhydroxid vom Typ Natriumhydroxid oder Kaliumhydroxid, (iv) einem Metallacetat vom Typ Zinkacetat oder Natriumacetat oder Kaliumacetat (v) einem Metalloxid (iv) oder einem Metallester oder (i) einem Metallester vom Typ Titanester.

6. Verfahren nach Anspruch 5, wobei der Katalysator ein Metallester ist, gewählt aus Titanester, Manganester, Antimonester oder Zinkacetat, Natriumacetat oder Kaliumacetat.

7. Verfahren nach einem der vorhergehenden Ansprüche, wobei der Katalysator in einem Molverhältnis von weniger als 35 % bezüglich des Polyesterterephthalats vorliegt.

8. Verfahren nach einem der vorhergehenden Ansprüche, wobei das polare Lösungsmittel einer der Formeln A oder B entspricht: 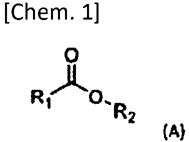 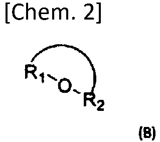 wobei R₁ und R₂ gleich oder unterschiedlich sind und aus einem Aryl-CₙH₂ₙ, Alkyl CₙH₂ₙ₊₁ oder CₙH₂ₙ₋₁ gewählt sind, mit n = 1 bis 10.

9. Verfahren nach Anspruch 8, wobei der Ester aus Methyl-, Ethyl-, Propyl-, Butyl-, Isopropyl- und Glykolacetat gewählt ist.

10. Verfahren nach einem der vorhergehenden Ansprüche, wobei das Verhältnis von Polyesterterephthalat zu Lösungsmittel zwischen 1:1,5 und 1:10 liegt.

11. Verfahren nach einem der vorhergehenden Ansprüche, wobei der Monoalkohol aus Methanol, Ethanol, Propanol oder Butanol gewählt ist und das Diol Ethylenglykol ist.

12. Verfahren nach einem der vorhergehenden Ansprüche, wobei das Molverhältnis (Äquivalent) von Alkohol zu Polyester-Terephthalat zwischen 0,25 und 16 liegt.

13. Verfahren nach einem der vorhergehenden Ansprüche, wobei für dieselbe Depolymerisationsreaktion ein Alkohol und ein Ester desselben Ranges verwendet werden.

## Claims

1. A method for recycling a material comprising terephthalate polyester into terephthalate diester including two steps:
a. step of milling or shredding said material to produce fragments, and
b. a step of depolymerizing said polyester into terephthalate ester in the presence of:
(i) a catalyst selected from a metal or organic ether oxide base, a metal acetate, a metal oxide, a metal hydroxide, a metal ester or a metal carbonate,
(ii) a polar solvent of the cyclic ester or ether oxide type
(iii) an alcohol selected from a monoalcohol or a diol
**characterized in that**:
- said base is present in catalytic amount relative to the amount of said polyester
- said depolymerization step is carried out at ambient temperature or by heating up to 70°C for a period of between 1 minute and 4 hours.

2. The method according to claim 1, wherein said material is composed of 100% terephthalate polyester selected from polyethylene terephthalate (PET) and polybutylene terephthalate (PBT).

3. The method according to claim 1, wherein said material is a composite material comprising terephthalate polyester selected from polyethylene terephthalate and polybutylene terephthalate blended with another component selected from cotton, polyamide, polyurethanes, polyolefins and fluorinated polymers.

4. The method according to claim 3, wherein said polyurethane is elastane.

5. The method according to one of claims 1 to 4, wherein said catalyst is selected from (i) an ether oxide is of the sodium methoxide, potassium methoxide, magnesium methoxide or ammonium methoxide type (ii) a metal carbonate of sodium carbonate or potassium carbonate type, (iii) a metal hydroxide of sodium hydroxide or potassium hydroxide type, (iv) a metal acetate of zinc acetate or sodium acetate or potassium acetate type (v) a metal oxide (iv) or a metal ester or (i) a metal ester of titanium ester type.

6. The method according to claim 5, wherein said catalyst is a metal ester selected from titanium ester, manganese ester, antimony ester, or zinc acetate, sodium acetate or potassium acetate.

7. The method according to one of the preceding claims, wherein said catalyst is present in a molar ratio of less than 35% relative to the terephthalate polyester.

8. The method according to one of the preceding claims, wherein said polar solvent satisfies one of formulas A or B: 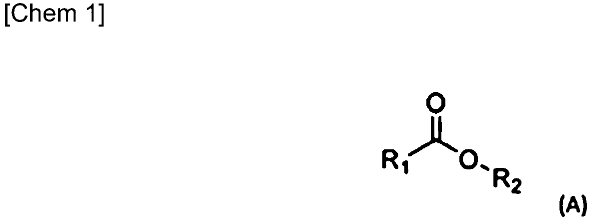 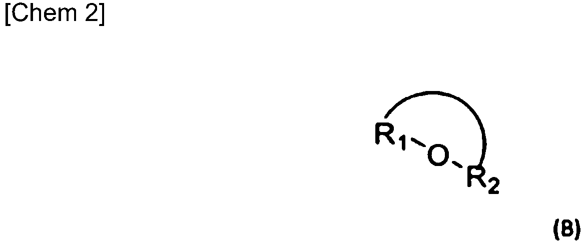 wherein R₁ and R₂ are identical or different and are selected from an aryl CₙH₂ₙ, alkyl CₙH₂ₙ₊₁ or CₙH₂ₙ₋₁ with n = 1 to 10.

9. The method according to claim 8, wherein said ester is selected from methyl acetate, ethyl acetate, propyl, butyl, isopropyl, glycol.

10. The method according to one of the preceding claims, wherein the terephthalate polyester:solvent ratio is between 1:1.5 and 1:10.

11. The method according to one of the preceding claims, wherein said monoalcohol is selected from methanol, ethanol, propanol or butanol and said diol is ethylene glycol.

12. The method according to one of the preceding claims, wherein the alcohol:terephthalate polyester molar ratio (equivalent) is comprised between 0.25 and 16.

13. The method according to one of the preceding claims, wherein an alcohol and an ester of the same rank are used for the same depolymerization reaction.
